(19) Europäisches Patentamt
European Patent Office
Office européen des brevets

(11) Publication number: **0 515 159 B1**

(12) # EUROPEAN PATENT SPECIFICATION

(45) Date of publication of patent specification: **29.11.95**

(51) Int. Cl.[6]: **C07C 69/80**, C07C 69/00, A61K 31/215, C07C 69/34

(21) Application number: **92304554.6**

(22) Date of filing: **20.05.92**

(54) **Ursane derivatives, their preparation and pharmaceutical formulations thereof.**

(30) Priority: **21.05.91 GB 9110929**

(43) Date of publication of application:
**25.11.92 Bulletin 92/48**

(45) Publication of the grant of the patent:
**29.11.95 Bulletin 95/48**

(84) Designated Contracting States:
**AT BE CH DE DK ES FR GB GR IT LI LU MC NL PT SE**

(56) References cited:
**EP-A- 0 100 516**
**DE-A- 2 412 355**
**DE-A- 2 452 501**

(73) Proprietor: **IDB HOLDING S.p.A.**
**Via Ripamonti, 99**
**I-20141 Milano (IT)**

(72) Inventor: **Nizzola Rita, IDB Holding Spa**
**Via Ripamonti, 99**
**I-20141 Milano (IT)**
Inventor: **Malandrino Salvatore, IDB Holding Spa**
**Via Ripamonti, 99**
**I-20141 Milano (IT)**
Inventor: **Pifferi Giorgio, IDB Holding Spa**
**Via Ripamonti, 99**
**I-20141 Milano (IT)**

(74) Representative: **Ritter, Stephen David et al**
**Mathys & Squire**
**100 Grays Inn Road**
**London WC1X 8AL (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel ursane derivatives, to methods for their preparation and to pharmaceutical formulations comprising the novel ursane derivatives.

It is known that various compounds of the triterpene type possess antiulcer activity. For example in the oleanane series, the sodium salt of the hemisuccinate of β-glycyrrhetic acid (carbenoxolone) has been used in the treatment of gastric and duodenal ulcers. However, its use is limited by the side effects caused by its mineral-corticoid activity (pseudoaldosteronism) (R. Doll et al., **Gut,** 9, 42, 1968). Because of this, changes have been made to the structure of carbenoxolone with a view to finding new semisynthetic derivatives free of side effects and with improved pharmacological effects. In particular, a series of derivatives without the 11-oxo function and containing the olean-12-ene nucleus are claimed to have valuable anti-ulcer properties and fewer side effects (**Chem Pharm. Bull. (Tokyo)** 37 2500, 1989).

EP 0 100 516 (and its United States equivalent US 4,530,934) and DE-2412355 disclose derivatives of ursane-28-carboxylic acid which possess *inter alia* antiulcer activity. DE 2452501 discloses erythrodiol derivatives and their pharmacological use as antiflammatory agents.

Surprisingly, we have found that certain compounds containing the ursane skeleton, have better anti-ulcer properties than the known derivatives of the oleanane series used hitherto and exhibit no mineral-corticoid activity.

According to one aspect of the present invention there are provided esters of urs-12-ene-3β,28-diol or ursa-9(11),12-diene-3β,28-diol having the formula

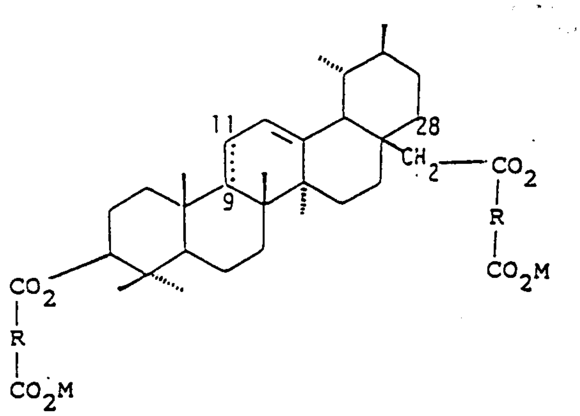

(I)
Ia=9,11-dihydro
Ib=Δ9(11)

in which the dotted line between carbon atoms 9 and 11 represents an optional bond, each of the structures -OC-R-CO- (which may be the same or different) represents the residue of a dicarboxylic acid having up to 12 carbon atoms and each M (which may be the same or different) represents hydrogen or one equivalent of a pharmaceutically acceptable inorganic or organic cation.

By "one equivalent of a pharmaceutically acceptable inorganic or organic cation" is meant an amount of the cation required for salt formation with a single $-COO^-$ group, i.e. 1/n atoms for a cation of valency n.

Examples of the pharmaceutically acceptable inorganic or organic cations include cations of alkali metals (sodium, potassium), alkaline earth metals (magnesium, calcium), and metals of Groups IIb, IIIb and Vb of the Periodic Table (zinc, aluminium, bismuth) and cations derived from basic organic compounds, for example amino acids containing basic groups capable of salt formation. Non-limiting examples of specific salts with amino acids containing basic groups include the salts of ornithine and lysine.

The structure -OC-R-CO- may be the residue of an aliphatic or aromatic dicarboxylic acid of the formula HOOC-R-COOH wherein R is an aliphatic or aromatic hydrocarbyl group containing 1 to 10 carbon atoms, for example an aliphatic dicarboxylic acid $HOOC-(CH_2)_n-COOH$ wherein n is 1 to 10, such as succinic acid or a residue of an aromatic dicarboxylic acid such as phthalic acid, isophthalic acid or terephthalic acid.

Most preferably -OC-R-CO- represents a residue of phthalic acid.

Thus according to a preferred aspect of the invention there are provided compounds of the formula

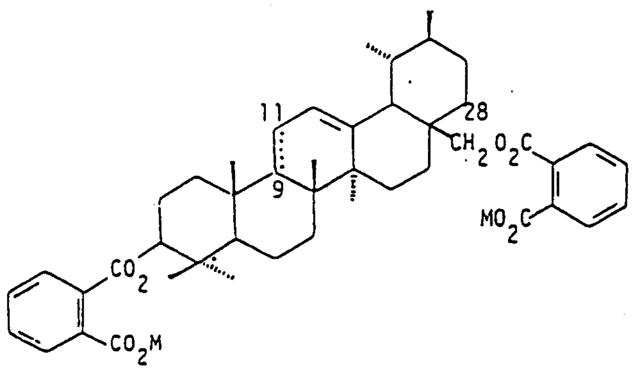

(II)
IIa=9,11-dihydro
IIb=Δ9(11)

in which the dotted line between carbon atoms 9 and 11 represents an optional bond, and each M represents hydrogen or one equivalent of a pharmaceutically acceptable inorganic or organic cation.

In the above formulae, when the the dotted line between carbon atoms 9 and 11 represents the presence of no additional bond between carbon atoms 9 and 11, i.e. when carbon atoms 9 and 11 are saturated, the above compounds of formulae I and II are esters of urs-12-ene-3β,28-diol.

Conversely, when the the dotted line between carbon atoms 9 and 11 represents an additional bond, i.e. when carbon atoms 9 and 11 are unsaturated, the above compounds of formulae I and II are esters of ursa-9(11),12-diene-3β,28-diol.

The preferred compounds of the present invention comprise the dihemiphthalate of urs-12-ene-3β,28-diol (IIa, M = H), the dihemiphthalate of ursa-9(11),12-diene-3β,28-diol (IIb, M = H) and their salts as mentioned above.

The compounds of the invention may be prepared by esterifying urs-12-ene-3β,28-diol (IIIa), otherwise known as uvaol with an appropriate dicarboxylic acid of formula HOOC-R-COOH, wherein R is as defined above, or an ester-forming derivative thereof, followed by optional steps, including introduction of an optional bond between carbon atoms 9 and 11, converting a free acid to a salt and converting a salt to a free acid.

Alternatively compound IIb may be prepared by esterifying ursa-9(11),12-diene-3β,28-diol (IIIb) with an appropriate dicarboxylic acid of formula HOOC-R-COOH, wherein R is as defined above or an ester-forming derivative thereof, followed by optional steps, including converting a free acid to a salt and converting a salt to a free acid.

Thus, as shown in the following scheme, compound IIa (M = H) may be prepared by esterification of urs-12-ene-3β,28-diol or uvaol (IIIa) with phthalic anhydride in a polar aprotic solvent, for example pyridine. Uvaol (IIIa) can be obtained by extraction from plants or by reduction with aluminium hydrides of ursolic acid (**Helv. Chim. Acta,** 37, 2145, 1954).

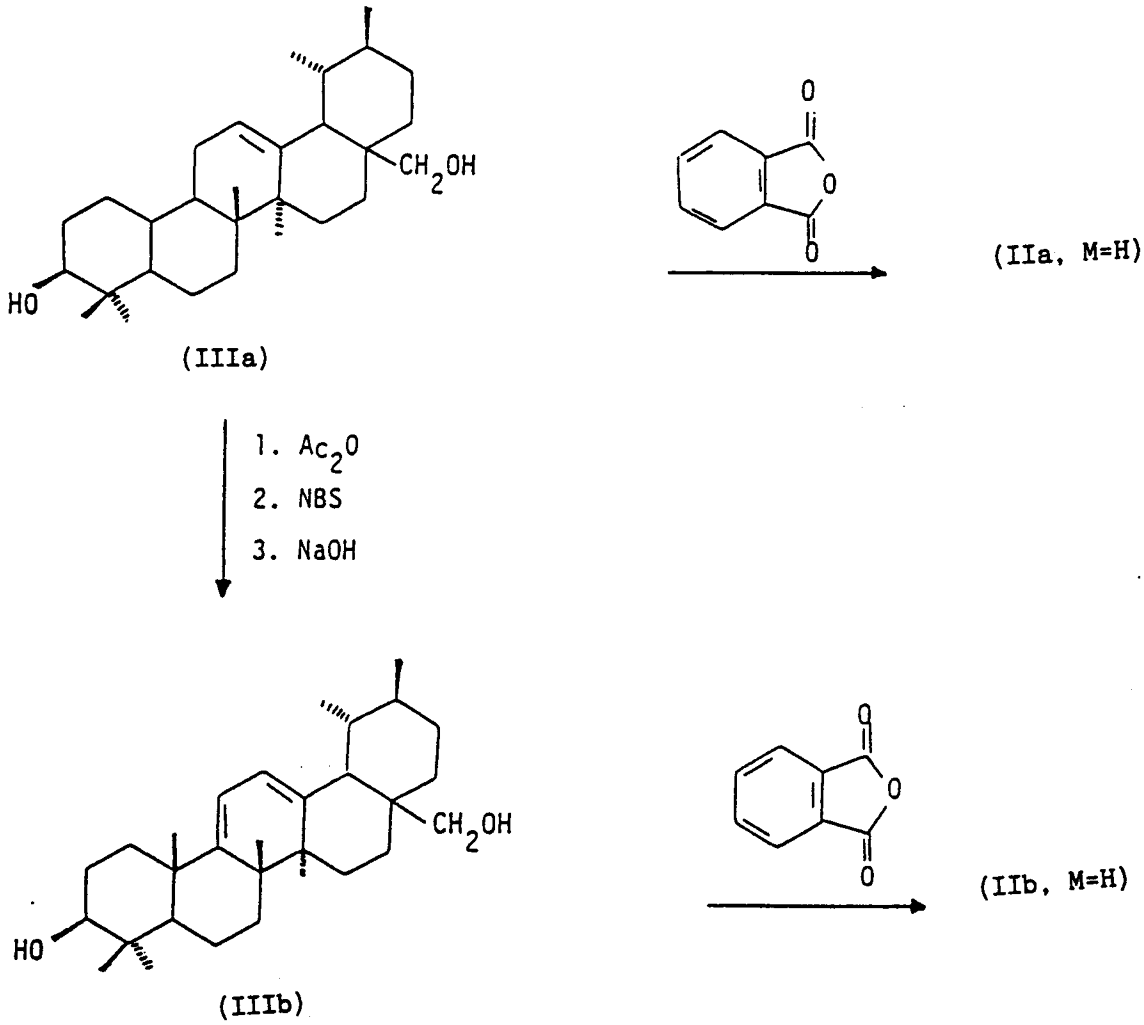


(IIIa)

(IIa, M=H)

(IIIb)

(IIb, M=H)

As shown in the above scheme, uvaol may also be used as the starting product for the synthesis of compound IIb (M = H). In these case, compound IIIa may be converted into the corresponding diacetate and brominated in position 11 by reacting it with N-bromosuccinimide (NBS). The brominated intermediate product is treated directly with alcoholic potassium hydroxide to give ursa-9(11),12-diene-3$\beta$,28-diol (IIIb), which is then esterified with phthalic anhydride to give IIb (M = H).

The acids IIa and IIb (M = H) can then be converted into salt by treatment with the stoichiometric quantity of the chosen base. For example, the corresponding disodium salts are obtained by treatment with sodium hydroxide in aqueous acetone solution. The raw sodium salts are precipitated and then recrystallised from alcohols or mixtures thereof.

The invention further provides pharmaceutical composition comprising an ester of urs-12-ene-3$\beta$,28-diol or ursa-9(11),12-diene-3$\beta$,28-diol having the formula

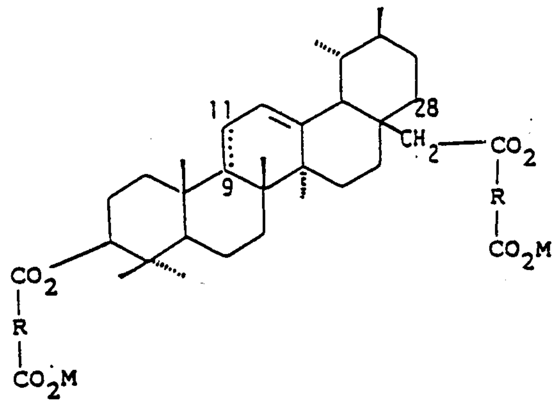

(I)
Ia=9,11-dihydro
Ib=Δ9(11)

in which the dotted line between carbon atoms 9 and 11 represents an optional bond, each of the structures -OC-R-CO- (which may be the same or different) represents the residue of a dicarboxylic acid having up to 12 carbon atoms and each M (which may be the same or different) represents hydrogen or one equivalent of a pharmaceutically acceptable inorganic or organic cation, and a pharmaceutically acceptable excipient.

Most preferably the pharmaceutical compositions of the invention comprise one of the preferred compounds of formula

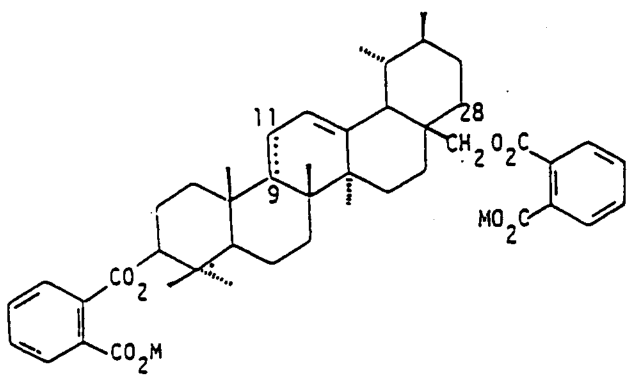

(II)
IIa=9,11-dihydro
IIb=Δ9(11)

in which the dotted line between carbon atoms 9 and 11 represents an optional bond, and each M (which may be the same or different) represents hydrogen or one equivalent of a pharmaceutically acceptable inorganic or organic cation.

Suitable dosage forms include tablets, capsules, suspensions, solutions and generally comprise any suitable pharmaceutically acceptable diluent or carrier.

The following examples serve to illustrate the invention without limiting its scope.

**EXAMPLE 1 Synthesis of the disodium salt of the dihemiphthalate or urs-12-ene-3β,28-diol (IIa, M=Na)**

A solution of 15 g urs-12-ene-3β,28-diol (IIIa) in 150 ml pyridine is treated with 20 g phthalic anhydride and heated to reflux for 16 hours. After this has cooled to room temperature and the reaction product has been precipitated by dilution with dilute hydrochloric acid, the precipitate is filtered off, washed with hot water and dried under vacuum.

This gives 22.8 g (yield 91%) dihemiphthalate of urs-12-ene-3β,28-diol (IIa, M = H): m.p. 142-144 °C $M^-$at 738 m/z.

The hemiester obtained is dissolved in acetone and treated with 59 ml 1N NaOH. The solid precipitated is filtered off, dissolved in methanol and diluted with isopropyl alcohol.

After concentration, 21 g (yield 89%) of IIa (M = Na) crystalline out: m.p. 250-253 °C.

| | | | |
|---|---|---|---|
| Elementary analysis: | C, 70.32; | H, 7.23; | Na, 5.81% |
| $C_{46}H_{56}Na_2O_8$ requires: | C, 70.57; | H, 7.21; | Na, 5.87% |

**EXAMPLE 2 Synthesis of ursa-9(11),12-diene-3β,28-diol (IIIb)**

To a solution of 50 g IIIa in anhydrous pyridine is added acetic anhydride as described in **Helv. Chim. Acta** 37, 2145 (1954). 52 g IIIa diacetate are obtained, which are treated under reflux for 2 hours in 5 l carbon tetrachloride with 21 g N-bromosuccinimide. After this has cooled to room temperature, the succinimide precipitate is filtered off and the organic phase washed with an aqueous solution of sodium metabisulphite.

The organic phase is evaporated to dryness and the residue treated under reflux for one hour with 5 l ethanol containing 25% of a concentrated aqueous solution of sodium hydroxide. After cooling to room temperature, this is diluted with water and the solid precipitated is crystallised from ethyl acetate. 36 g (73%) IIIb are obtained: m.p. 245-7° C, $M^+$ at 440 m/z.

| | | |
|---|---|---|
| Elementary analysis: | C, 81.64; | H, 11.04% |
| $C_{30}H_{48}O_2$ requires: | C, 81.76; | H, 10.98% |

**EXAMPLE 3 Synthesis of the disodium salt of the dihemiphthalate of ursa-9(11),12-diene-3β,28-diol (IIb, M=Na)**

A solution of 30 g ursa-9(11),12-diene-3β,28-diol (IIIb) in 25 ml pyridine is treated under reflux for 24 hours with 40 g phthalic anhydride. After cooling to room temperature and dilution with dilute hydrochloric acid, the precipitate is filtered off, washed with hot water and dried under vacuum. 44 g (yield 89%) dihemiphthalate of ursa-9(11),12-diene-3β,28-diol (IIb, M=H) are obtained: m.p. 138-140°C and $M^-$ at 736 m/z.

The hemiester obtained is dissolved in acetone and treated with 58 ml 1N NaOH. The solid precipitated is filtered off, dissolved in methanol and diluted with isopropyl alcohol. After concentration, 40 g IIb (M=Na) (yield 90%) crystallise out: m.p. 214-216 °C.

| | | | |
|---|---|---|---|
| Elementary analysis: | C, 70.49; | H, 7.01; | Na, 5,87% |
| $C_{46}H_{54}Na_2O_8$ requires: | C, 70.75; | H, 6.97; | Na, 5.89% |

**Example 4 Synthesis of the L-lysine salt of the dihemiphthalate of urs-12-ene-3β,28-diol (IIa, M=$^+NH_3(CH_2)_4CH(NH_2)CO_2H$)**

A solution of 7.38 g IIa (M=H), obtained as described in Example 1, in 50 ml methanol is treated with an aqueous solution containing 2.92 g L-lysine. This is stirred until dissolution is complete, is concentrated under vacuum until the methanol is eliminated and freeze-dried. 10.3 g IIa are obtained (as lysine salt).

| | | | |
|---|---|---|---|
| Elementary analysis: | C, 67.21; | H, 8.42; | N, 5.37% |
| $C_{58}H_{88}N_4O_{12}$ requires: | C, 67.44; | H, 8.53; | N, 5.43% |

PHARMACOLOGICAL ACTIVITY

The antiulcer activity of the disodium salts of compounds Ia and Ib was evaluated in two experimental ulcer models in the rat.

### 1) Alcohol-induced ulcer

The gastric lesions were induced in male Sprague-Dawley rats weighing 120-140 g, which had been fasted for 48 h, by administering 1 ml/rat of 90% alcohol by mouth. One hour after administration of the damage-inducing agent, the animals were sacrifed in an excess of ether and the stomachs opened along the major curvature; the gastric lesions were measured in $mm^2$ and expressed as the percentage of inhibition compared with the controls. The substances under investigation, suspended in 0.5% Methocel, were administered by mouth 1 hour before the alcohol.

The results shown in the following Table 1 illustrate that compounds IIa and IIb (M = Na) are capable of inhibiting the alcohol-induced lesions on a dose-related basis, with $ED_{50}$ values of 3.7 (1.4-9.4) and 16.8 (9.8-28.9) mg/kg by mouth respectively.

By way of comparison, carbenoxolone was found to have an $ED_{50}$ of 90.4 (38.5-212.6) mg/kg by mouth and 3β-(carboxypropionyloxy)ursa-9(11),12dien-28-oic acid (a compound disclosed in US-A-4 530 934 and corresponding DE-A-2 412 355) had an $ED_{50}$ of 106.5 (54.0-210.2) mg/kg by mouth.

TABLE 1

| Effects on alcohol-induced gastric lesions in the rat | | | | |
|---|---|---|---|---|
| Substances | Dose mg/kg os | % Inhibition | $ED_{50}$ mg/kg and confidence limits (p = 0.95) | R |
| IIa (M = Na) | 2.0<br>2.5<br>3.125<br>6.25 | 16.1<br>22.1<br>65.2<br>67.4 | 3.7 (1.4-9.4) | 25.6 (12.6-52.0) |
| IIb (M = Na) | 6.25<br>12.5<br>16.0<br>25.0 | 10.7<br>32.5<br>38.2<br>76.6 | 16.8 (9.8-28.9) | 5.5 (2.7-11.4) |
| 18β-olean-12-ene-3β,30-diol di-O-hemiphthalate | 0.8<br>3.12<br>12.5<br>50.0 | 12.0<br>32.0<br>40.8<br>70.2 | 14.6 (6.1-35.3) | 5.6 (1.4-23.5) |
| Carbenoxolone | 50.0<br>100.0<br>200.0 | 34.9<br>45.0<br>83.0 | 90.4 (38.5-212.6) | 1.0 |
| 3β-(carboxypropionyloxy)-ursa-9(11),-12-dien-28-oic acid | 50.0<br>100.0<br>200.0<br>400.0 | 28.4<br>45.6<br>76.3<br>80.1 | 106.5 (54.0-210.2) | 0.8 (0.4-1.5) |

### 2) Diclofenac-induced ulcer

Female Sprague-Dawley rats weighing 180-200 g that had been fasted for 24 h were used in this experiment. The gastric lesions were induced by a single oral dose of 100 mg/kg Diclofenac.

The animals were sacrificed 5 h after administration of the damage-inducing agent. The gastric lesions were measured in $mm^2$ and expressed as a percentage of inhibition.

The substances under investigation, suspended in 0.5% Methocel, were administered by mouth 1 h before the anti-inflammatory agent.

The results shown in Table 2 indicate that compounds IIa and IIb (M = Na) inhibit the lesions induced by the anti-inflammatory agent on a dose-related basis, with $ED_{50}$ values of 7.0 (4.1-11.8) and 6.4 (2.4-16.6) mg/kg by mouth respectively. Carbenoxolon has an $ED_{50}$ of 140.2 (87.2-225.3) mg/kg by mouth in this experimental model.

TABLE 2

| **Effects on Diclofenac-induced gastric ulcers in the rat** | | | | |
|---|---|---|---|---|
| Substances | Dose mg/kg os | % Inhibition | $ED_{50}$ and confidence limits (p = 0.95) | R |
| IIa (M = Na) | 3.125 | 8.9 | 7.0 (4.1-11.8) | 16.3 (8.8-30.0) |
| | 6.25 | 28.3 | | |
| | 7.5 | 62.3 | | |
| | 9.0 | 67.9 | | |
| IIb (M = Na) | 3.125 | 37.4 | 6.4 (2.4-16.6) | 17.8 (10.2-31.3) |
| | 6.25 | 43.3 | | |
| | 12.5 | 72.0 | | |
| | 25.0 | 73.3 | | |
| 18β-olean-12-ene-3β,30-diol di-O-hemiphthalate | 3.125 | 1.5 | 38.9 (21.8-69.6) | 3.5 (1.5-8.0) |
| | 6.25 | 5.9 | | |
| | 12.5 | 21.5 | | |
| | 25.0 | 29.8 | | |
| | 50.0 | | | |
| Carbenoxolone | 100.0 | 37.2 | 140.2 (87.2-225.3) | 1.0 |
| | 150.0 | 45.6 | | |
| | 200.0 | 72.7 | | |
| | 400.0 | 90.9 | | |

**3) Antidiuretic activity**

The effect of compounds IIa and IIb (M = Na) on urinary excretion was investigated in male Sprague-Dawley rats weighing 180-200 g. The animals were treated with the compounds under investigation in a dose of 20 mg/kg once a day for 5 days. Carbenoxolone in a dose of 200 mg/kg was used as a reference substance. The animals were fasted for 18 h before the last dose. The urine was collected for 6 hours after administration of the fifth dose and its content of $Na^+$ and $K^+$ was measured. The results shown in the following Table 3:

TABLE 3

**Effects on urinary excretion in the rat**

| Substances | Dose mg/kg os | Volume urine (0.6 h) ml ± s.d. | Electrolyte output, mEq/6h/rat $Na^+$ | $K^+$ | $Na^+/K^+$ |
|---|---|---|---|---|---|
| Controls | - | 3.3±0.3 | 0.22±0.01 | 0.13±0.01 | 1.7 |
| IIa (M = Na) | 20 | 3.0±0.2 | 0.21±0.01 | 0.14±0.01 | 1.5 |
| IIb (M = Na) | 20 | 2.8±0.3 | 0.19±0.02 | 0.13±0.02 | 1.5 |
| Carbenoxolone | 200 | 1.5±0.2** | 0.10±0.01** | 0.12±0.01 | 0.8 |

** $p < 0.01$ Dunnett t test

The results shown in the above Table 3 indicate that the administration of these compounds does not induce any significant changes in the urinary parameters.

By contrast, carbenoxolone causes a significant reduction in the excretion of $Na^+$ (72%), thereby reducing diuresis (67%) and the $NA^+/K^+$ ratio (from 1.7 to 0.5).

**4) Acute toxicity**

The acute toxicity of compounds IIa and IIb (M = Na) was evaluated in the rat after oral and intraperitoneal administration.

Each experimental group comprised 5 male and 5 female Sprague-Dawley rats. After being treated, the animals were kept under observation for 14 days and the dose that caused the death of 50% of the animals ($LD_{50}$) was calculated by the probit method (Finney D .J. in "Probit Analysis", Cambridge University Press, 3rd Ed., Cambridge, 1971). The results shown in Table 4 indicate that, after intraperitoneal administration, the toxicity of both compounds is similar to that of the reference product.

TABLE 4

**Acute toxicity in the rat**

| Compounds | $LD_{50}$ (confidence limits p = 0.95) mg/kg | |
|---|---|---|
| | i.p | p.o |
| IIa (M = Na) | 60 (44 - 82) | >1000 ($LD_{10}$)* |
| IIb (M = Na) | 75 (57 - 99) | >1000 ($LD_{10}$)* |
| Carbenoxolone | 92 (80 - 106) | 3100 (2750-3820) |

* Maximum dose that could be administered (2 doses of 500 mg/kg 1 h apart)

After oral administration, both compounds have an $LD_{50}$ of 1000 mg/kg ($LD_{10}$). It was not possible to administer higher doses since the rheological properties of more concentrated suspensions prevent them from passing through the gastric tube.

It is clear from the results reported here that the ursane derivatives IIa and IIb exhibit significantly greater antiulcer activity than carbenoxolone, which is an oleanane derivative. The calculation of the potency ratio (R, Tables 1 and 2) shows that both compounds are much more potent than the reference drugs.

In addition, compounds IIa and IIb in their effective doses have no adverse effects on sodium retention. By contrast, carbenoxolone is known to produce such side effects even in the rat.

Comparison of the $LD_{50}$ and $ED_{50}$ values, as shown in Table 5, reveals that the compounds under investigation have a far more favourable therapeutic index than carbenoxolone.

TABLE 5

| Comparison of the oral therapeutic indices ($LD_{50}/ED_{50}$) | | |
|---|---|---|
| Compound | Alcohol-induced ulcer | Diclofenac-induced ulcer |
| IIa (M = Na) | > 270 | > 143 |
| IIb (M = Na) | > 60 | > 156 |
| Carbenoxolone | 34 | 22 |

On the basis of these results, the compounds that form the subject of the present invention exhibit powerful antiulcer activity with virtually no adverse effects on sodium retention in the pharmacologically active doses. These compounds are therefore promising new therapeutic agents for the treatment of gastric and duodenal ulcer when administered by mouth in appropriate formulations.

The following examples of pharmaceutical formulations are given as examples and do not limit the scope of the invention.

**Capsules**

| | |
|---|---|
| Compound IIa (M = Na) | 10 mg |
| Lactose | 158 mg |
| Talc | 10 mg |
| Magnesium stearate | 2 mg |

**Sachets**

| | |
|---|---|
| Compound IIa (M = Na) | 5 mg |
| Mannitol | 782 mg |
| Aluminium hydroxide | 350 mg |
| Magnesium trisilicate | 200 mg |
| Lactose | 100 mg |
| Flavouring | 40 mg |
| Polyvinylpyrrolidone | 20 mg |
| Ammonium glycyrrhizinate | 3 mg |

**Syrup**

100 ml syrup contain:

| | |
|---|---|
| Compound IIa (M = Na) | 25 mg |
| Sorbitol (70%), non-crystallisable | 20 g |
| Hydroxyethylcellulose | 0.4 g |
| Glycerol | 5 g |
| Flavouring | 0.2 g |
| Methyl p-hydroxybenzoate | 0.1 g |
| Propyl p-hydroxybenzoate | 0.02 g |
| Sodium saccharine | 0.02 g |
| Purified water, q.b. to | 100 ml |

## Claims

### Claims for the following Contracting States : AT, BE, CH, LI, DE, DK, FR, GB, IT, LU, MC, NL, PT, SE

**1.** An ester of urs-12-en-3$\beta$,28-diol or ursa-9(11),12-dien-3$\beta$,28-diol having the formula

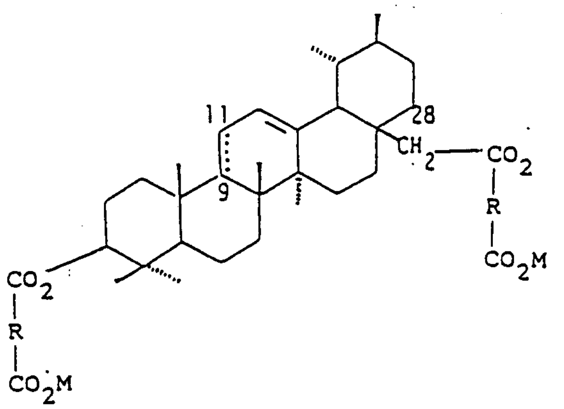

(I)
Ia=9,11-dihydro
Ib=Δ9(11)

in which the dotted line between carbon atoms 9 and 11 represents an optional bond, each of the structures -OC-R-CO- (which may be the same or different) represents the residue of a dicarboxylic acid having up to 12 carbon atoms and each M (which may be the same or different) represents hydrogen or one equivalent of a pharmaceutically acceptable inorganic or organic cation.

**2.** A compound according to Claim 1 wherein the pharmaceutically acceptable inorganic or organic cations are selected from cations of alkali metals (sodium, potassium), alkaline earth metals, and metals of Groups IIb, IIIb and Vb of the Periodic Table (zinc, aluminium, bismuth) and cations derived from basic organic compounds, for example amino acids containing basic groups capable of salt formation.

**3.** A compound according to Claim 2 wherein the amino acid is ornithine or lysine.

**4.** A compound according to any preceding claim wherein structure -OC-R-CO- is a residue of an aliphatic or aromatic dicarboxylic acid of the formula HOOC-R-COOH wherein R is an aliphatic or aromatic hydrocarbyl group containing 1 to 10 carbon atoms.

**5.** A compound according to any preceding claim wherein structure -OC-R-CO- is a residue of phthalic acid.

**6.** A compound of the formula

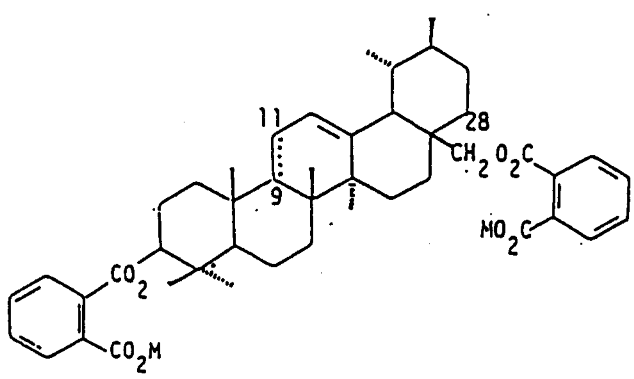

(II)
IIa=9,11-dihydro
IIb=Δ9(11)

in which the dotted line between carbon atoms 9 and 11 represents an optional bond, and each M represents hydrogen or one equivalent of a pharmaceutically acceptable inorganic or organic cation.

7. A compound according to Claim 6 wherein the pharmaceutically acceptable inorganic or organic cations are selected from cations of alkali metals (sodium, potassium), alkaline earth metals, and metals of Groups IIb, IIIb and Vb of the Periodic Table (zinc, aluminium, bismuth) and cations derived from basic organic compounds, for example amino acids containing basic groups capable of salt formation.

8. A compound according to Claim 7 wherein the amino acid is ornithine or lysine.

9. A process for producing a compound according to any preceding claim which comprises
(i) esterifying urs-12-en-3β,28-diol (IIIa), with an appropriate dicarboxylic acid of formula HOOC-R-COOH, wherein R is as defined in Claim 4, or an ester-forming derivative thereof, followed by optional steps, including introduction of an optional bond between carbon atoms 9 and 11, converting a free acid to a salt and converting a salt to a free acid, or
(ii) esterifying urs-9(11)12-dien-3β,28-diol (IIIb) with an appropriate dicarboxylic acid of formula HOOC-R-COOH, wherein R is as defined in Claim 4 or an ester-forming derivative thereof, followed by optional steps, including converting the double bond between carbon atoms 9 and 11 to a single bond, converting a free acid to a salt and converting a salt to a free acid.

10. A pharmaceutical composition comprising an ester of urs-12-en-3β,28-diol or ursa-9(11),12-dien-3β,28-diol having the formula

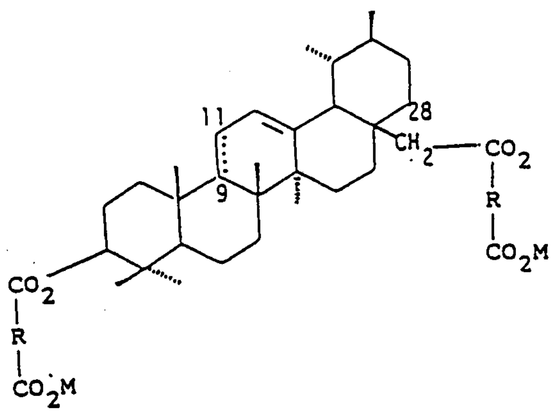

(I)
Ia=9,11-dihydro
Ib=Δ9(11)

in which the dotted line between carbon atoms 9 and 11 represents an optional bond, each of the structures -OC-R-CO- (which may be the same or different) represents the residue of a dicarboxylic acid having up to 12 carbon atoms and each M (which may be the same or different) represents hydrogen or one equivalent of a pharmaceutically acceptable inorganic or organic cation, and a pharmaceutically acceptable excipient.

11. A pharmaceutical composition according to Claim 10 wherein the compound of formula I is as defined in any of Claims 2 to 5

**12.** The use of an ester of urs-12-en-3$\beta$,28-diol or ursa-9(11),12-dien-3$\beta$,28-diol having the formula

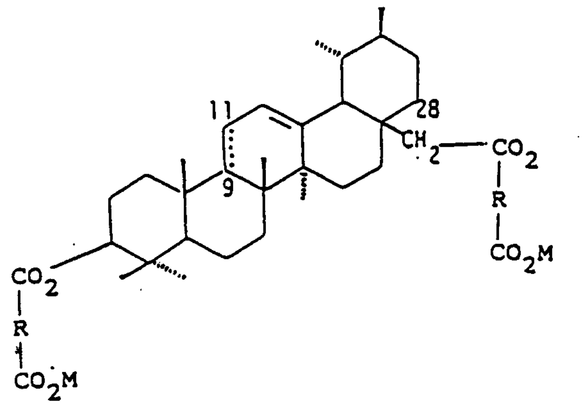

(I)
Ia=9,11-dihydro
Ib=$\Delta$9(11)

in which the dotted line between carbon atoms 9 and 11 represents an optional bond, each of the structures -OC-R-CO- (which may be the same or different) represents the residue of a dicarboxylic acid having up to 12 carbon atoms and each M (which may be the same or different) represents hydrogen or one equivalent of a pharmaceutically acceptable inorganic or organic cation in the manufacture of a pharmaceutical composition for producing an anti-ulcer effect in a subject.

**Claims for the following Contracting States : ES, GR**

**1.** A process for producing an ester of urs-12-en-3$\beta$,28-diol or ursa-9(11),12-dien-3$\beta$,28-diol having the formula

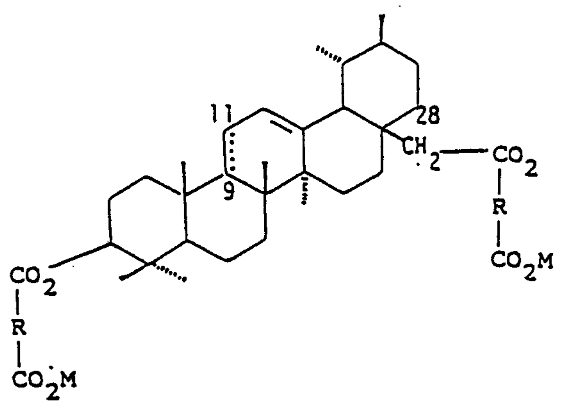

(I)
Ia=9,11-dihydro
Ib=$\Delta$9(11)

in which the dotted line between carbon atoms 9 and 11 represents an optional bond, each of the structures -OC-R-CO- (which may be the same or different) represents the residue of a dicarboxylic acid having up to 12 carbon atoms and each M (which may be the same or different) represents hydrogen or one equivalent of a pharmaceutically acceptable inorganic or organic cation, which comprises

(i) esterifying urs-12-en-3$\beta$,28-diol (IIIa), with an appropriate dicarboxylic acid of formula HOOC-R-COOH, wherein R is as defined above, or an ester-forming derivative thereof, followed by optional steps, including introduction of an optional bond between carbon atoms 9 and 11, converting a free acid to a salt and converting a salt to a free acid, or

(ii) esterifying urs-9(11)12-dien-3$\beta$,28-diol (IIIb) with an appropriate dicarboxylic acid of formula HOOC-R-COOH, wherein R is as defined in above or an ester-forming derivative thereof, followed by optional steps, including converting the double bond between carbon atoms 9 and 11 to a single bond, converting a free acid to a salt and converting a salt to a free acid.

**2.** A process according to Claim 1 wherein the pharmaceutically acceptable inorganic or organic cations are selected from cations of alkali metals (sodium, potassium), alkaline earth metals, and metals of

Groups IIb, IIIb and Vb of the Periodic Table (zinc, aluminium, bismuth) and cations derived from basic organic compounds, for example amino acids containing basic groups capable of salt formation.

**3.** A process according to Claim 2 wherein the amino acid is ornithine or lysine.

**4.** A process according to any preceding claim wherein structure -OC-R-CO- is a residue of an aliphatic or aromatic dicarboxylic acid of the formula HOOC-R-COOH wherein R is an aliphatic or aromatic hydrocarbyl group containing 1 to 10 carbon atoms.

**5.** A process according to any preceding claim wherein structure -OC-R-CO- is a residue of phthalic acid.

**6.** A process according to Claim 1 wherein the produced compound has the formula

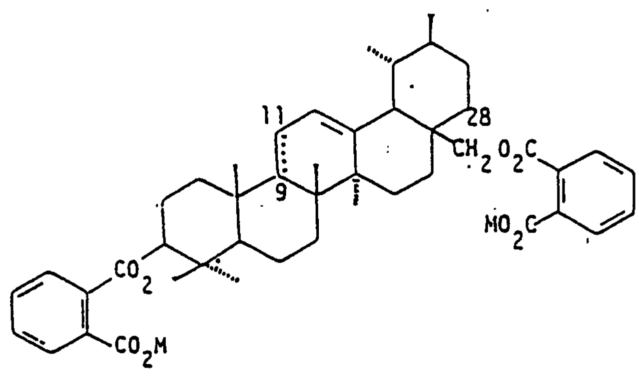

(II)
IIa=9,11-dihydro
IIb=Δ9(11)

in which the dotted line between carbon atoms 9 and 11 represents an optional bond, and each M represents hydrogen or one equivalent of a pharmaceutically acceptable inorganic or organic cation.

**7.** A process according to Claim 6 wherein the pharmaceutically acceptable inorganic or organic cations are selected from cations of alkali metals (sodium, potassium), alkaline earth metals, and metals of Groups IIb, IIIb and Vb of the Periodic Table (zinc, aluminium, bismuth) and cations derived from basic organic compounds, for example amino acids containing basic groups capable of salt formation.

**8.** A process according to Claim 7 wherein the amino acid is ornithine or lysine.

**9.** A process for producing a pharmaceutical composition which comprises admixing an ester of urs-12-en-3β,28-diol or ursa-9(11),12-dien-3β,28-diol having the formula

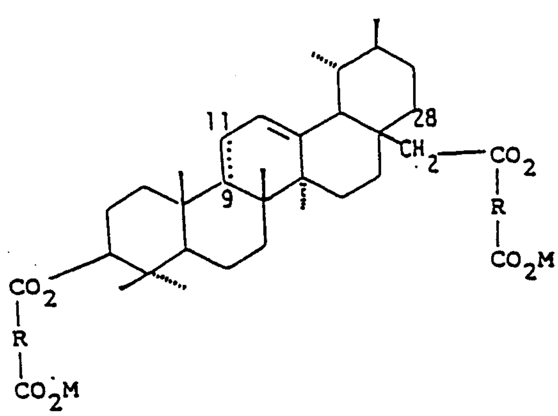

(I)
Ia=9,11-dihydro
Ib=Δ9(11)

in which the dotted line between carbon atoms 9 and 11 represents an optional bond, each of the structures -OC-R-CO- (which may be the same or different) represents the residue of a dicarboxylic acid having up to 12 carbon atoms and each M (which may be the same or different) represents

hydrogen or one equivalent of a pharmaceutically acceptable inorganic or organic cation, with a pharmaceutically acceptable excipient.

10. A process according to Claim 9 wherein the compound of formula I is as defined in any of Claims 2 to 5

11. The use of an ester of urs-12-en-3$\beta$,28-diol or ursa-9(11),12-dien-3$\beta$,28-diol having the formula

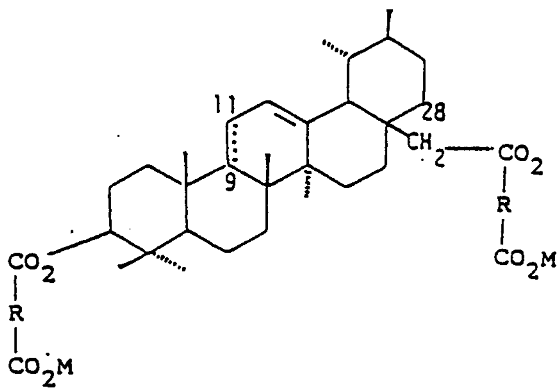

(I)
Ia=9,11-dihydro
Ib=Δ9(11)

in which the dotted line between carbon atoms 9 and 11 represents an optional bond, each of the structures -OC-R-CO- (which may be the same or different) represents the residue of a dicarboxylic acid having up to 12 carbon atoms and each M (which may be the same or different) represents hydrogen or one equivalent of a pharmaceutically acceptable inorganic or organic cation in the manufacture of a pharmaceutical composition for producing an anti-ulcer effect in a subject.

**Patentansprüche**

**Patentansprüche für folgende Vertragsstaaten : AT, BE, CH, LI, DE, DK, FR, GB, IT, LU, MC, NL, PT, SE**

1. 3$\beta$,28-Urs-12-endiol- oder 3$\beta$,28-Urs-9(11),12-diendiolester der Formel:

**(I)**
**Ia = 9,11-dihydro**
**Ib = Δ9(11)**

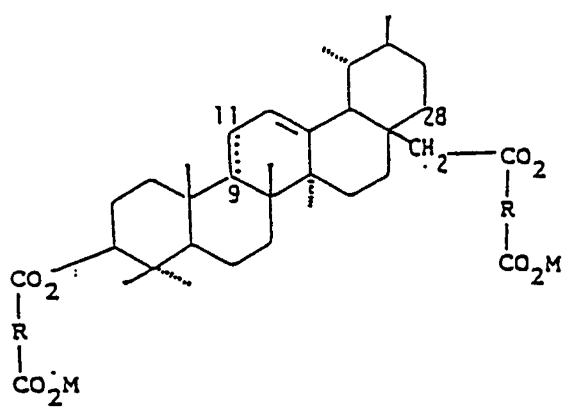

in der

die gepunktete Linie zwischen den Kohlenstoffatomen 9 und 11 eine gegebenenfalls vorhandene Bindung bedeutet,

jede der Strukturen -OC-R-CO- (die gleich oder verschieden sein können) einen Dicarbonsäurerest mit bis zu 12 Kohlenstoffatomen darstellt und

jedes M (die gleich oder verschieden sein können) Wasserstoff oder ein Äquivalent eines pharmazeutisch geeigneten anorganischen oder organischen Kations darstellt.

2. Verbindung gemaß Anspruch 1, in der die pharmazeutisch geeigneten anorganischen oder organischen Kationen aus Kationen der Alkalimetalle (Natrium, Kalium), der Erdalkalimetalle und der Metalle der Gruppe IIb, IIIb und Vb des Periodensystems (Zink, Aluminium, Wismut) sowie aus von basischen organischen Verbindungen abgeleiteten Kationen, beispielsweise aus basischen, zur Salzbildung befähigte Gruppen enthaltenden Aminosäuren, ausgewählt sind.

3. Verbindung gemaß Anspruch 2, in der die Aminosäure Ornithin oder Lysin ist.

4. Verbindung gemaß einem der vorstehenden Ansprüche, in der die Struktur

   -OC-R-CO-

   den Rest einer aliphatischen oder aromatischen Dicarbonsäure der Formel HOOC-R-COOH darstellt, in der R ein aliphatischer oder aromatischer Kohlenwasserstoffrest mit 1 bis 10 Kohlenstoffatomen ist.

5. Verbindung gemäß einem der vorstehenden Ansprüche, in der die Struktur -OC-R-CO- ein Phthalsäurerest ist.

6. Verbindung der Formel

   (II)

   IIa = 9,11-dihydro

   IIb = Δ9(11)

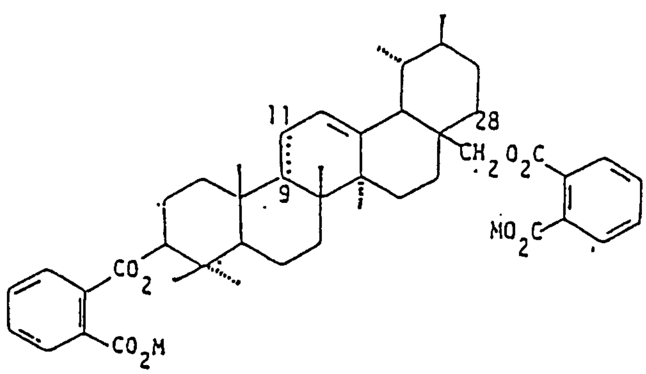

   in der

   die gepunktete Linie zwischen den Kohlenstoffatomen 9 und 11 eine gegebenenfalls vorhandene Bindung bedeutet und

   jedes M Wasserstoff oder ein Äquivalent eines pharmazeutisch geeigneten anorganischen oder organischen Kations darstellt.

7. Verbindung gemaß Anspruch 6, in der die pharmazeutisch geeigneten anorganischen oder organischen Kationen aus Kationen der Alkalimetalle (Natrium, Kalium), der Erdalkalimetalle und der Metalle der Gruppe IIb, IIIb und Vb des Periodensystems (Zink, Aluminium, Wismut) sowie aus von basischen organischen Verbindungen abgeleiteten Kationen, beispielsweise aus basischen, zur Salzbildung befähigte Gruppen enthaltenden Aminosäuren, ausgewählt sind.

8. Verbindung gemaß Anspruch 7, in der die Aminosäure Ornithin oder Lysin ist.

9. Verfahren zur Herstellung einer Verbindung gemaß einem der vorstehenden Ansprüche, bei dem man

   (i) 3β,28-Urs-12-endiol (IIIa) mit einer geeigneten Dicarbonsäure der Formel HOOC-R-COOH, in der R wie in Anspruch 4 definiert ist, oder einem esterbildenden Derivat derselben verestert, wahlweise gefolgt von Einführen einer möglichen Bindung zwischen den Koblenstoffatomen 9 und 11, Umwandeln der freien Säure in ein Salz oder Umwandeln des Salzes in die freie Säure oder

   (ii) 3β,28-Urs-9(11),12-diendiol (IIIb) mit einer geeigneten Dicarbonsäure der Formel HOOC-R-COOH, in der R wie in Anspruch 4 definiert ist, oder einem esterbildenden Derivat derselben verestert, wahlweise gefolgt von Umwandlung der Doppelbindung zwischen den Kohlenstoffatomen 9 und 11 in eine Einfachbindung, Umwandeln der freien Säure in ein Salz oder Umwandeln des Salzes in die freie Säure.

**10.** Arzneimittel, enthaltend einen 3β,28-Urs-12-endiol- oder 3β,28-Urs-9(11),12-diendiolester der Formel:

(I)

Ia = 9,11-dihydro

Ib = Δ9(11)

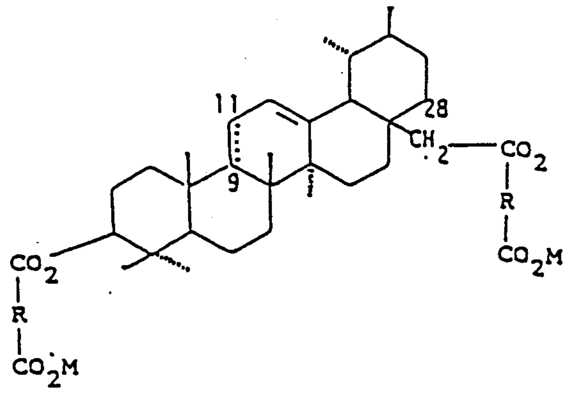

in der

die gepunktete Linie zwischen den Kohlenstoffatomen 9 und 11 eine gegebenenfalls vorhandene Bindung bedeutet,

jede der Strukturen -OC-R-CO- (die gleich oder verschieden sein können) einen Dicarbonsäurerest mit bis zu 12 Kohlenstoffatomen darstellt und

jedes M (die gleich oder verschieden sein können) Wasserstoff oder ein Äquivalent eines pharmazeutisch geeigneten anorganischen oder organischen Kations darstellt,

sowie ein pharmazeutisch geeignetes Excipiens.

**11.** Arzneimittel gemäß Anspruch 10, bei dem die Verbindung der Formel (I) die in einem der Ansprüche 2 bis 5 angegebene Bedeutung hat.

**12.** Verwendung eines 3β,28-Urs-12-endiol- oder 3β,28-Urs-9(11),12-diendiolesters der Formel:

(I)

Ia = 9,11-dihydro

Ib = Δ9(11)

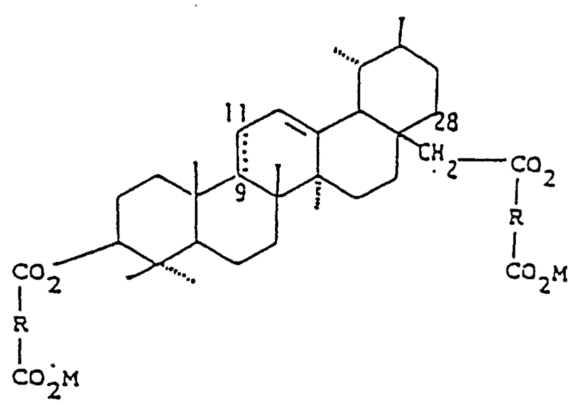

in der

die gepunktete Linie zwischen den Kohlenstoffatomen 9 und 11 eine gegebenenfalls vorhandene Bindung bedeutet,

jede der Strukturen -OC-R-CO- (die gleich oder verschieden sein können) einen Dicarbonsäurerest mit bis zu 12 Kohlenstoffatomen darstellt und

jedes M (die gleich oder verschieden sein können) Wasserstoff oder ein Äquivalent eines pharmazeutisch geeigneten anorganischen oder organischen Kations darstellt,

zur Herstellung eines Arzneimittels mit anti-ulcer-Wirkung beim Menschen.

## Patentansprüche für folgende Vertragsstaaten : ES, GR

**1.** Verfahren zur Herstellung eines 3β,28-Urs-12-endiol- oder 3β,28-Urs-9(11),12-diendiolesters der Formel:

(I)

Ia = 9,11-dihydro

Ib = Δ9(11)

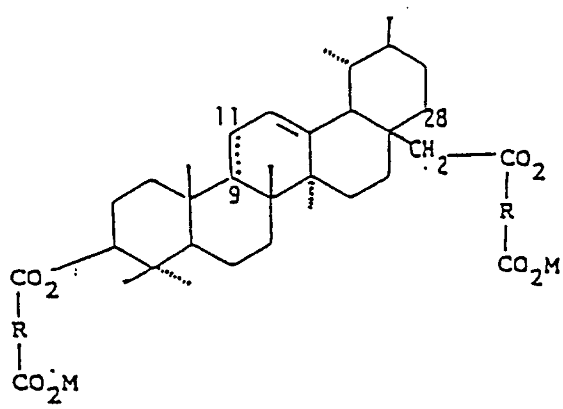

in der

die gepunktete Linie zwischen den Kohlenstoffatomen 9 und 11 eine gegebenenfalls vorhandene Bindung bedeutet,

jede der Strukturen -OC-R-CO- (die gleich oder verschieden sein können) einen Dicarbonsäurerest mit bis zu 12 Kohlenstoffatomen darstellt und

jedes M (die gleich oder verschieden sein können) Wasserstoff oder ein Äquivalent eines pharmazeutisch geeigneten anorganischen oder organischen Kations darstellt, bei dem man

(i) 3β,28-Urs-12-endiol (IIIa) mit einer geeigneten Dicarbonsäure der Formel HOOC-R-COOH, in der R wie in Anspruch 4 definiert ist, oder einem esterbildenden Derivat derselben verestert, wahlweise gefolgt von Einführen einer möglichen Bindung zwischen den Kohlenstoffatomen 9 und 11, Umwandeln der freien Säure in ein Salz oder Umwandeln des Salzes in die freie Säure oder

(ii) 3β,28-Urs-9(11),12-diendiol (IIIb) mit einer geeigneten Dicarbonsäure der Formel HOOC-R-COOH, in der R wie in Anspruch 4 definiert ist, oder einem esterbildenden Derivat derselben verestert, wahlweise gefolgt von Umwandlung der Doppelbindung zwischen den Kohlenstoffatomen 9 und 11 in eine Einfachbindung, Umwandeln der freien Säure in ein Salz oder Umwandeln des Salzes in die freie Säure.

2. Verfahren gemäß Anspruch 1, bei dem die pharmazeutisch geeigneten anorganischen oder organischen Kationen aus Kationen der Alkalimetalle (Natrium, Kalium), der Erdalkalimetalle und der Metalle der Gruppe IIb, IIIb und Vb des Periodensystems (Zink, Aluminium, Wismut) sowie aus von basischen organischen Verbindungen abgeleiteten Kationen, beispielsweise aus basischen, zur Salzbildung befähigte Gruppen enthaltenden Aminosäuren, ausgewählt sind.

3. Verfahren gemäß Anspruch 2, bei dem die Aminosäure Ornithin oder Lysin ist.

4. Verfahren gemaß einem der vorstehenden Ansprüche, bei dem die Struktur -OC-R-CO den Rest einer aliphatischen oder aromatischen Dicarbonsäure der Formel HOOC-R-COOH darstellt, in der R ein aliphatischer oder aromatischer Kohlenwasserstoffrest mit 1 bis 10 Kohlenstoffatomen ist.

5. Verfahren gemäß einem der vorstehenden Ansprüche, bei dem die Struktur -OC-R-CO- ein Phthalsäurerest ist.

**6.** Verfahren gemäß Anspruch 1, bei dem das erzeugte Produkt der Formel

(II)

IIa = 9,11-dihydro

IIb = Δ9(11)

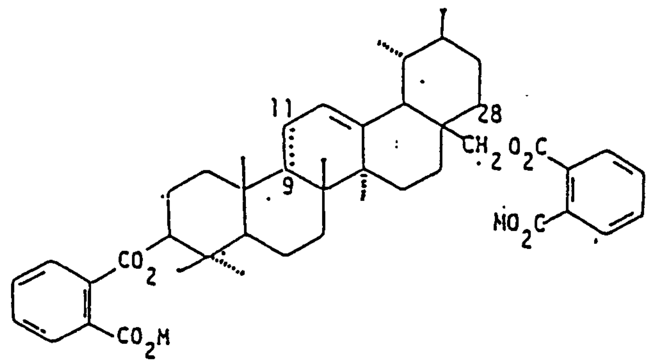

entspricht, in der
die gepunktete Linie zwischen den Kohlenstoffatomen 9 und 11 eine gegebenenfalls vorhandene Bindung bedeutet und
jedes M Wasserstoff oder ein Äquivalent eines pharmazeutisch geeigneten anorganischen oder organischen Kations darstellt.

**7.** Verfahren gemäß Anspruch 6, in der die pharmazeutisch geeigneten anorganischen oder organischen Kationen aus Kationen der Alkalimetalle (Natrium, Kalium), der Erdalkalimetalle und der Metalle der Gruppe IIb, IIIb und Vb des Periodensystems (Zink, Aluminium, Wismut) sowie aus von basischen organischen Verbindungen abgeleiteten Kationen, beispielsweise aus basischen, zur Salzbildung befähigte Gruppen enthaltenden Aminosäuren, ausgewählt sind.

**8.** Verfahren gemaß Anspruch 7, in der die Aminosäure Ornithin oder Lysin ist.

**9.** Verfahren zur Herstellung eines Arzneimittels, bei dem man
einen 3β,28-Urs-12-endiol- oder 3β,28-Urs-9(11),12-diendiolester der Formel

(I)

Ia = 9,11-dihydro

Ib = Δ9(11)

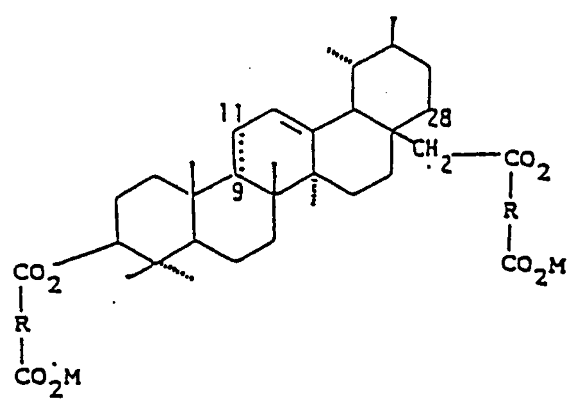

in der
die gepunktete Linie zwischen den Kohlenstoffatomen 9 und 11 eine gegebenenfalls vorhandene Bindung bedeutet,
jede der Strukturen -OC-R-CO- (die gleich oder verschieden sein können) einen Dicarbonsäurerest mit bis zu 12 Kohlenstoffatomen darstellt und
jedes M (die gleich oder verschieden sein können) Wasserstoff oder ein Äquivalent eines pharmazeutisch geeigneten anorganischen oder organischen Kations darstellt,
mit einem pharmazeutisch geeigneten Excipiens umsetzt.

**10.** Verfahren gemaß Anspruch 9, bei dem die Verbindung der Formel (I) die in einem der Ansprüche 2 bis 5 angegebene Bedeutung hat.

**11.** Verwendung eines 3β,28-Urs-12-endiol- oder 3β,28-Urs-9(11),12-diendiolesters der Formel:

(I)

Ia = 9,11-dihydro

Ib = Δ9(11)

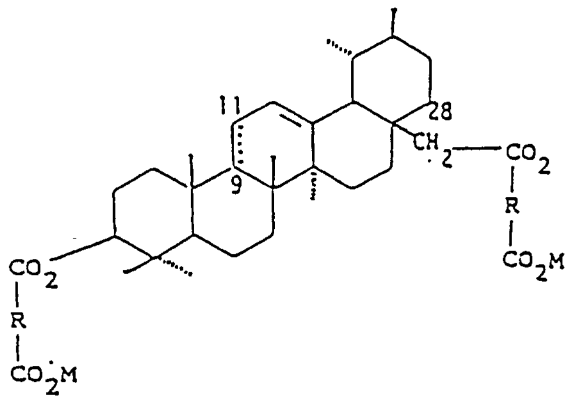

in der

die gepunktete Linie zwischen den Kohlenstoffatomen 9 und 11 eine gegebenenfalls vorhandene Bindung bedeutet,

jede der Strukturen -OC-R-CO- (die gleich oder verschieden sein können) einen Dicarbonsäurerest mit bis zu 12 Kohlenstoffatomen darstellt und

jedes M (die gleich oder verschieden sein können) Wasserstoff oder ein Äquivalent eines pharmazeutisch geeigneten anorganischen oder organischen Kations darstellt,

zur Herstellung eines Arzneimittels mit anti-ulcer-Wirkung beim Menschen.

**Revendications**

**Revendications pour les Etats contractants suivants : AT, BE, CH, LI, DE, DK, FR, GB, IT, LU, MC, NL, PT, SE**

**1.** Ester d'urs-12-ène-3β,28-diol ou d'ursa-9(11),12-diène-3β,28-diol de formule :

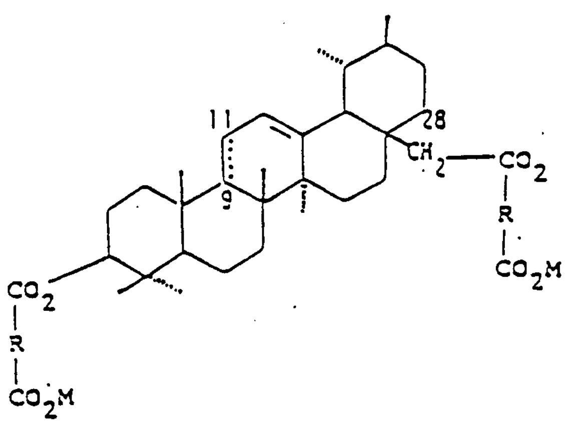

(I)

Ia=9.11-dihydro

Ib=Δ9(11)

dans laquelle la ligne interrrompue entre les atomes de carbone 9 et 11 représente une liaison éventuelle, chacune des structures -OC-R-CO- (qui peuvent être identiques ou différentes) représente le résidu d'un acide dicarboxylique comportant jusque 12 atomes de carbone, et chacun des groupes M (qui peuvent être identiques ou différents) représente un atome d'hydrogène, ou un équivalent d'un cation minéral ou organique, pharmaceutiquement acceptable.

**2.** Composé selon la revendication 1, dans lequel les cations minéraux ou organiques, pharmaceutiquement acceptables sont choisis parmi les cations de métaux alcalins (sodium, potassium), de métaux alcalinoterreux, et de métaux des Groupes IIb, IIIb, et Vb du Tableau Périodique (zinc, aluminium,

bismuth) et les cations dérivant de composés basiques, par exemple, les acides aminés contenant des groupes basiques capables de former des sels.

**3.** Composé selon la revendication 2, dans lequel l'acide aminé est l'ornithine ou la lysine.

**4.** Composé selon l'une quelconque des revendications précédentes, dans lequel la structure -OC-R-CO- est un résidu d'acide dicarboxylique aliphatique ou aromatique de formule HOOC-R-COOH, dans laquelle R est un groupe hydrocarbyle aliphatique ou aromatique comportant 1 à 10 atomes de carbone.

**5.** Composé selon l'une quelconque des revendications précédentes, dans lequel la structure -OC-R-CO- est un résidu d'acide phtalique.

**6.** Composé de formule :

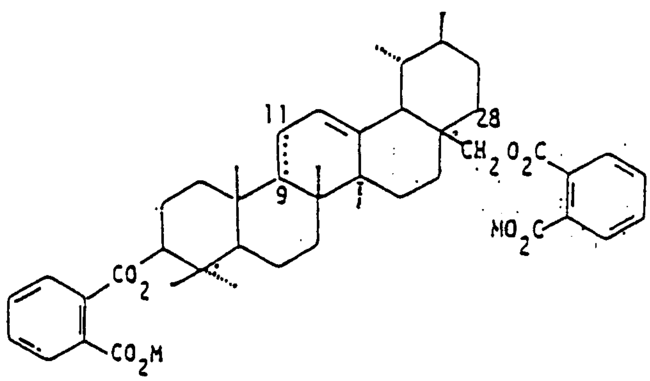

(II)
IIa=9,11-dihydro
IIb=Δ9(11)

dans laquelle la ligne interrrompue entre les atomes de carbone 9 et 11 représente une liaison éventuelle, et chacun des groupes M représente un atome d'hydrogène, ou un équivalent d'un cation minéral ou organique, pharmaceutiquement acceptable.

**7.** Composé selon la revendication 6, dans lequel les cations minéraux ou organiques, pharmaceutiquement acceptables, sont choisis parmi les cations de métaux alcalins (sodium, potassium), de métaux alcalinoterreux, et de métaux des Groupes IIb, IIIb, et Vb du Tableau Périodique (zinc, aluminium, bismuth) et les cations dérivant de composés basiques, par exemple, les acides aminés contenant des groupes basiques capables de former des sels.

**8.** Composé selon la revendication 7, dans lequel l'acide aminé est l'ornithine ou la lysine.

**9.** Procédé pour la production d'un composé selon l'une quelconque des revendications précédentes, qui comprend :

(i) l'estérification de l'urs-12-ène-3$\beta$,28-diol (IIIa), avec un acide dicarboxylique approprié de formule HOOC-R-COOH dans laquelle R est tel que défini dans le revendication 4, ou avec un dérivé de celui-ci, générateur d'ester, suivie d'étapes éventuelles comprenant l'introduction d'une liaison éventuelle entre les atomes de carbone 9 et 11, la conversion d'un acide libre en un sel, et la conversion d'un sel en acide libre,ou

(ii) l'estérification de l'urs-9(11),12-diène-3$\beta$,28-diol (IIIb) avec un acide dicarboxylique approprié de formule HOOC-R-COOH dans laquelle R est tel que défini dans le revendication 4, ou avec un dérivé de celui-ci, générateur d'ester, suivie d'étapes éventuelles comprenant la conversion de la double liaison entre les atomes de carbone 9 et 11 en une simple liaison, la conversion d'un acide libre en un sel, et la conversion d'un sel en acide libre.

**10.** Composition pharmaceutique comprenant un ester d'urs-12-ène-3$\beta$,28-diol ou d'ursa-9(11), 12-diène-3$\beta$,28-diol de formule :

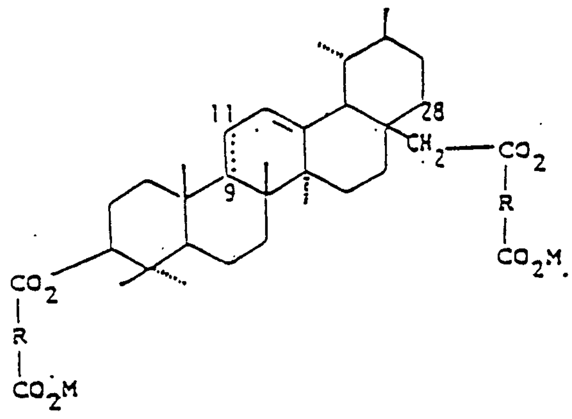

(I)
Ia=9,11-dihydro
Ib=Δ9(11)

dans laquelle la ligne interrrompue entre les atomes de carbone 9 et 11 représente une liaison éventuelle, chacune des structures -OC-R-CO- (qui peuvent être identiques ou différentes) représente le résidu d'un acide dicarboxylique comportant jusque 12 atomes de carbone, et chacun des groupes M (qui peuvent être identiques ou différents) représente un atome d'hydrogène, ou un équivalent d'un cation minéral ou organique, pharmaceutiquement acceptable, et un excipient pharmaceutiquement acceptable.

**11.** Composition pharmaceutique selon la revendication 10, dans laquelle le composé de formule I est tel que défini dans l'une quelconque des revendications 2 à 5.

**12.** Utilisation d'un ester d'urs-12-ène-3$\beta$,28-diol ou d'ursa-9(11),12-diène-3$\beta$,28-diol de formule :

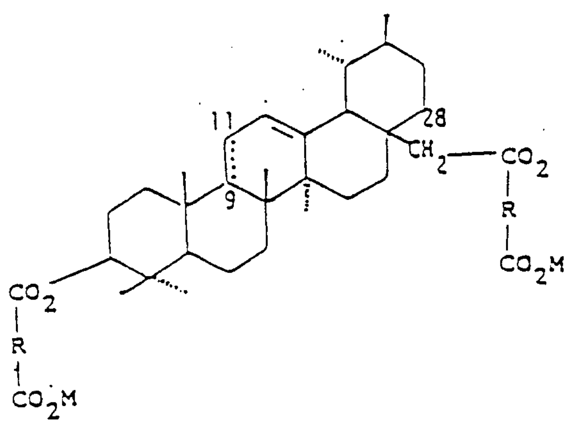

(I)
Ia=9,11-dihydro
Ib=Δ9(11)

dans laquelle la ligne interrrompue entre les atomes de carbone 9 et 11 représente une liaison éventuelle, chacune des structures -OC-R-CO- (qui peuvent être identiques ou différentes) représente le résidu d'un acide dicarboxylique comportant jusque 12 atomes de carbone, et chacun des groupes M (qui peuvent être identiques ou différents) représente un atome d'hydrogène, ou un équivalent d'un cation minéral ou organique, pharmaceutiquement acceptable, dans la fabrication d'une composition pharmaceutique pour la production d'un effet anti-ulcère chez un sujet.

**Revendications pour les Etats contractants suivants : ES, GR**

**1.** Procédé pour la production d'un ester d'urs-12-ène-3$\beta$, 28-diol ou d'ursa-9(11),12-diène-3$\beta$,28-diol de formule :

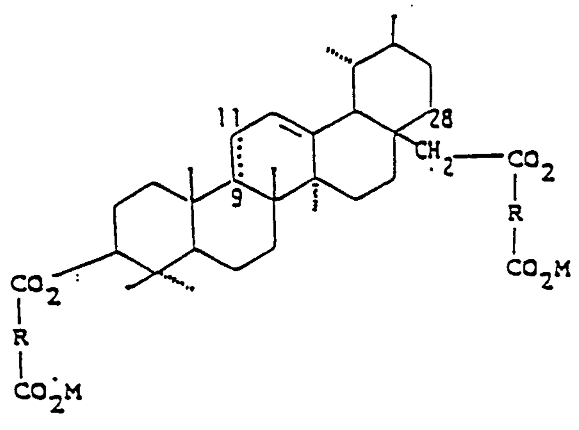

(I)

Ia=9,11-dihydro

Ib=Δ9(11)

dans laquelle la ligne interrrompue entre les atomes de carbone 9 et 11 représente une liaison éventuelle, chacune des structures -OC-R-CO- (qui peuvent être identiques ou différentes) représente le résidu d'un acide dicarboxylique comportant jusque 12 atomes de carbone, et chacun des groupes M (qui peuvent être identiques ou différents) représente un atome d'hydrogène, ou un équivalent d'un cation minéral ou organique, pharmaceutiquement acceptable, lequel procédé comprend :

(i) l'estérification de l'urs-12-ène-3β,28-diol (IIIa), avec un acide dicarboxylique approprié de formule HOOC-R-COOH dans laquelle R est tel que défini ci-dessus, ou avec un dérivé de celui-ci, générateur d'ester, suivie d'étapes éventuelles comprenant l'introduction d'une liaison éventuelle entre les atomes de carbone 9 et 11, la conversion d'un acide libre en un sel, et la conversion d'un sel en acide libre,ou

(ii) l'estérification de l'urs-9(11),12-diène-3β,28-diol (IIIb) avec un acide dicarboxylique approprié de formule HOOC-R-COOH dans laquelle R est tel que défini ci-dessus, ou avec un dérivé de celui-ci, générateur d'ester, suivie d'étapes éventuelles comprenant la conversion de la double liaison entre les atomes de carbone 9 et 11 en une simple liaison, la conversion d'un acide libre en un sel, et la conversion d'un sel en acide libre.

2. Procédé selon la revendication 1, dans lequel les cations minéraux ou organiques, pharmaceutiquement acceptables sont choisis parmi les cations de métaux alcalins (sodium, potassium), de métaux alcalinoterreux, et de métaux des Groupes IIb, IIIb, et Vb du Tableau Périodique (zinc, aluminium, bismuth) et les cations dérivant de composés basiques, par exemple; les acides aminés contenant des groupes basiques capables de former des sels.

3. Procédé selon la revendication 2, dans lequel l'acide aminé est l'ornithine ou la lysine.

4. Procédé selon l'une quelconque des revendications précédentes, dans lequel la structure -OC-R-CO- est un résidu d'acide dicarboxylique aliphatique ou aromatique de formule HOOC-R-COOH, dans laquelle R est un groupe hydrocarbyle aliphatique ou aromatique comportant 1 à 10 atomes de carbone.

5. Procédé selon l'une quelconque des revendications précédentes, dans lequel la structure -OC-R-CO- est un résidu d'acide phtalique.

6. Procédé selon la revendication 1, dans lequelle composé produit a la formule :

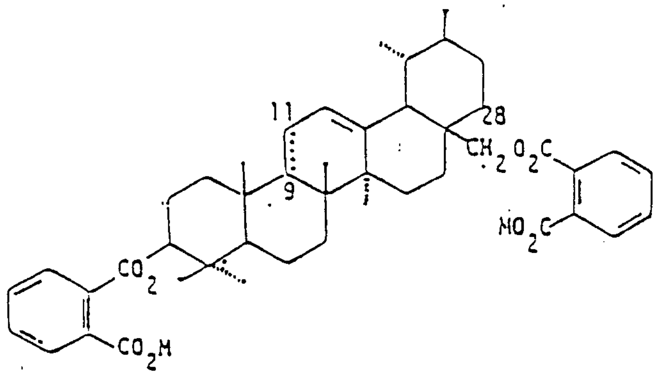

(II)
IIa=9,11-dihydro
IIb=Δ9(11)

dans laquelle la ligne interrrompue entre les atomes de carbone 9 et 11 représente une liaison éventuelle, et chacun des groupes M représente un atome d'hydrogène, ou un équivalent d'un cation minéral ou organique, pharmaceutiquement acceptable.

7. Procédé selon la revendication 6, dans lequel les cations minéraux ou organiques, pharmaceutiquement acceptables, sont choisis parmi les cations de métaux alcalins (sodium, potassium), de métaux alcalinoterreux, et de métaux des Groupes IIb, IIIb, et Vb du Tableau Périodique (zinc, aluminium, bismuth) et les cations dérivant de composés basiques, par exemple, les acides aminés contenant des groupes basiques capables de former des sels.

8. Procédé selon la revendication 7, dans lequel l'acide aminé est l'ornithine ou la lysine.

9. Procédé pour la production d'une composition pharmaceutique comprenant le mélange d'un ester d'urs-12-ène-3$\beta$,28-diol ou d'ursa-9(11 ),12-diène-3$\beta$,28-diol de formule :

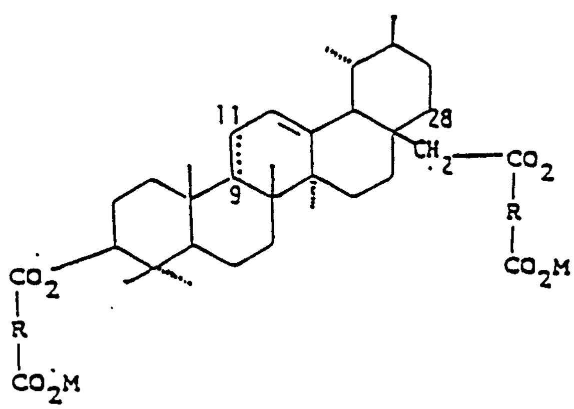

(I)
Ia=9,11-dihydro
Ib=Δ9(11)

dans laquelle la ligne interrrompue entre les atomes de carbone 9 et 11 représente une liaison éventuelle, chacune des structures -OC-R-CO- (qui peuvent être identiques ou différentes) représente le résidu d'un acide dicarboxylique comportant jusque 12 atomes de carbone, et chacun des groupes M (qui peuvent être identiques ou différents) représente un atome d'hydrogène, ou un équivalent d'un cation minéral ou organique, pharmaceutiquement acceptable, avec un excipient pharmaceutiquement acceptable.

10. Procédé selon la revendication 9, dans laquelle le composé de formule 1 est tel que défini dans l'une quelconque des revendications 2 à 5.

**11.** Utilisation d'un ester d'urs-12-ène-3β,28-diol ou d'ursa-9(11), 12-diène-3β,28-diol de formule :

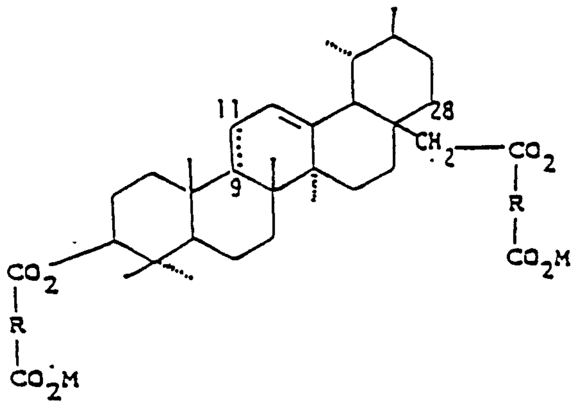

(I)
Ia=9.11-dihydro
Ib=Δ9(11)

dans laquelle la ligne interrrompue entre les atomes de carbone 9 et 11 représente une liaison éventuelle, chacune des structures -OC-R-CO- (qui peuvent être identiques ou différents) représente le résidu d'un acide dicarboxylique comportant jusque 12 atomes de carbone, et chacun des groupes M (qui peuvent être identiques ou différents) représente un atome d'hydrogène, ou un équivalent d'un cation minéral ou organique, pharmaceutiquement acceptable, dans la fabrication d'une composition pharmaceutique pour la production d'un effet anti-ulcère chez un sujet.